# EUROPEAN PATENT SPECIFICATION

(11) **EP 3 216 460 B1**
(45) Date of publication and mention of the grant of the patent: **20.11.2019**
(21) Application number: 15857030.9
(22) Date of filing: 07.08.2015
(51) Int. Cl.: A61K 39/385, G01N 33/53, C07K 14/71

(54) **ANTIGEN POLYPEPTIDE FOR DETECTING PLASMA IMMUNOLOGICAL MARKER-VEGFR1 AUTOANTIBODY AND USE THEREOF**
ANTIGENPOLYPEPTID FÜR DEN NACHWEIS VON IMMUNOLOGISCHEM MARKER-VEGFR1-AUTOANTIKÖRPER IN PLASMA UND VERWENDUNG
POLYPEPTIDE D'ANTIGÈNE POUR DÉTECTER UN AUTO-ANTICORPS CONTRE VEGFR1-MARQUEUR IMMUNOLOGIQUE PLASMATIQUE ET SON UTILISATION

(30) Priority: 04.11.2014 CN 201410615968
(43) Date of publication of application: 13.09.2017
(73) Proprietor: Qingdao Hailanshen Biotechnology Co., Ltd, Qingdao, Shandong 266031 (CN)
(72) Inventor: WEI, Jun, Shenzhen Guangdong 518052 (CN); WANG, Weili, Shenzhen Guangdong 518052 (CN)
(74) Representative: Hanna Moore + Curley
(86) International application number: PCT/CN2015/086348
(87) International publication number: WO 2016/070662

(56) References cited:
- CN-A- 102 134 277
- CN-A- 102 724 996
- CN-A- 104 356 226
- K YOSHIMURA ET AL: "Phase I clinical trial of human vascular endothelial growth factor receptor 1 peptide vaccines for patients with metastatic renal cell carcinoma", BRITISH JOURNAL OF CANCER, vol. 108, no. 6, 7 March 2013 (2013-03-07) , pages 1260-1266, XP055408106, GB ISSN: 0007-0920, DOI: 10.1038/bjc.2013.90
- ISHIZAKI, H. ET AL.: 'Inhibition of Tumor Growth with Antiangiogenic Cancer Vaccine Using Epitope Peptides Derived from Human Vascular Endothelial Growth Factor Receptor 1' CLIN CANCER RES vol. 12, no. 19, 01 October 2006, pages 5841 - 5849, XP055404119 DOI: 10.1158/1078-0432.CCR-06-0750
- CHEN, CHUAN ET AL.: 'Development of Anti-tumour Drugs Targeting VEGF/VEGFR: a Progress' CHINESE JOURNAL OF CANCER BIOTHERAPY vol. 14, no. 3, 30 June 2007, XP009500118

<110> Qingdao Hailanshen Biotechnology Co., Ltd.
<120> Antigen polypeptide for detecting plasma immune marker - VEGFR1 autoantibody and
   application thereof
<130> Invention
<160> 3
<170> Patentln version 3.3
<210> 1
   <211> 34
   <212> PRT
   <213> Sequence 1
<400> 1
   Asp Glu Gly Val Tyr His Cys Lys Ala Thr Asn Gln Lys Gly Ser Val
   1               5                   10                  15
   Glu Ser Ser Ala Tyr Leu Thr Val Gln Gly Thr Ser Asp Lys Ser Asn
               20                  25                  30
   Leu Glu
<210> 2
   <211> 33
   <212> PRT
   <213> Sequence 2
<400> 2
   Asp Leu Lys Leu Ser Cys Thr Val Asn Lys Phe Leu Tyr Arg Asp Val
   1               5                   10                  15
   Thr Trp Ile Leu Leu Arg Thr Val Asn Asn Arg Thr Met His Tyr Ser
               20                  25                  30
   Ile
<210> 3
   <211> 34
   <212> PRT
   <213> Sequence 3
<400> 3
   Glu Ser Gly Leu Ser Asp Val Ser Arg Pro Ser Phe Cys His Ser Ser
   1 5 10 15
   Cys Gly His Val Ser Glu Gly Lys Arg Arg Phe Thr Tyr Asp His Ala
   20 25 30
   Glu Leu

## Description

### TECHNICAL FIELD

The present invention belongs to the technical field of immunology, and relates to a VEGFR1 antigen polypeptide. The antigen polypeptide can be used to prepare a specific reagent for detecting VEGFR1 antibody level in human plasma.

### BACKGROUND ART

The recent studies have shown that discovering and determining tumor-associated antibody in blood of healthy people has the value of instructing targeted treatment. For example, Trastuzumab and Bevacizumab which have been broadly used in the clinic take epidermal growth factor receptor-2 (HER2) and vascular endothelial growth factor A (VEGF-A) as targets, which become an importance means of the clinical treatment for advanced stage tumor. However, since the side effects are too great, these two monoclonal antibodies can only be used as third line treatment medicine. Thus, developing a new means used for preventing tumor metastasis and recurrence after local treatment while having low side effects is a present problem urgently needed to solve. But in the field of immune treatment, the lead technical barrier for solving this problem is to find an innovative practical solution for target technology, then a brand new tumor immune treatment solution can be further developed.

VEGFR1 is also referred to as vascular endothelial growth factor receptor-1, and has been considered as one of specific proteins expressed by cancer cells. A large number of studies have found that many human solid tumors can express VEGFR1 abundantly, such as liver cancer, lung cancer, kidney cancer, intestinal cancer, breast cancer and so on. Because VEGFR1 antigen epitope polypeptide is mainly located on the surface of tumor cell and can be used as target spot for corresponding antibody killing tumor, the medical field universally acknowledges that the clinical application of corresponding antibody of VEGFR1 is very likely to become a brand new anticancer treatment means which is achieved fastest and has low side effects. Yoshimura et al, "Phase I clinical trial of human vascular endothelial growth factor receptor 1 peptide vaccines for patients with metastatic renal cell carcinoma", British Journal of Cancer (2013) 108,1260-1266 is a general background study evaluating the safety of vascular endothelial growth factor receptor 1 (VEGFR1) peptide vaccination in metastatic renal cell carcinoma (mRCC) patients. The results of this study concluded that the VEGFR1 peptide vaccine is safe, well-tolerated and recommended for further trials with mRCC patients. ISHIZAKI, H. et al., "Inhibition of Tumor Growth with Antiangiogenic Cancer Vaccine Using Epitope Peptides Derived from Human Vascular Endothelial Growth Factor Receptor 1", CLIN CANCER RES, (2006), vol. 12, no. 19 is directed to a study of the inhibition of tumor growth using specific epitope peptides derived from human vascular endothelial growth factor receptor 1 (VEGFR1).

Currently, in the field of biotechnology, the only way is to carry out the detection and screening of corresponding antibody of VEGFR1 using recombinant protein as antigen, which will go through vector construction, transfection, expression, screening, purifying and other tedious processes. However, since the space structure of protein is complicated and linear antigen epitope is hardly to be exposed, specificity of the antibody detected by using recombinant protein is poor, and a considerable proportion of the antibody cannot be combined with antigen target spot on the surface of tumor cell membrane, as a result, only substandard qualitative detection means can be carried out, and the practical means for tumor prevention and treatment cannot be developed accordingly. Meanwhile, high sensitivity of ELISA method has an extreme high requirement for stability of protein purification technology, therefore, the recombinant protein meeting the requirement of detection is costly. There is as yet no effective means for detection and screening of the corresponding antibody of VEGFR1 at home and abroad.

The linear VEGFR1 antigen epitope polypeptide specifically designed by the present invention can effectively solve the above-mentioned technical problems, prepare detection reagent with extremely high specificity, detect the index value of concentration of the immune marker - VEGFR1 antibody in the plasma of healthy blood donors according to set process specification, and obtain package solutions for the detection, identification, qualitative and quantitative applications of VEGFR1 antibody with high accuracy, simple operation and moderate cost. Thus, the technology of the present invention can lay a realistic basis for developing a brand new tumor immune treatment solution with low side effects using natural VEGFR1 antibodies in healthy human body.

### SUMMARY OF THE INVENTION

The object of the present invention is to provide a linear antigen polypeptide for detecting VEGFR1 autoantibody, and thus to prepare a specific reagent for detecting VEGFR1 autoantibody, and design a corresponding preferably implemented operation process scheme. The present invention as defined in claim 1 as a composition for detecting VEGFGR1 autoantibody, comprising three VEGFR1 linear antigen polypeptides, whose amino acid sequences are shown in Table 1.

**Table 1. VEGFR1 antigen polypeptide sequence**

| | |
|---|---|
| Abbreviations VEGFR1 | |

It is well known that, since the combination of antigen with antibody actually only occurs between the antigenic determinant and antibody binding site.

More fully complementary space structure and configuration of antigen and antibody lead to more stable binding of the antigen and the antibody, stronger specificity and higher binding efficiency. Since a target antibody and a binding site structure thereof are prerequisites, the binding state and affinity characteristics of the whole protein antigen and the antibody can be determined according to the antigenic determinant.

Accordingly, based on the biological characteristics of VEFGR1 protein, the immunoinformatics forecast and simulation regarding to multiple epitopes, and analysis of various parameters related to antigenicity, the present invention as defined in claim 1 designs the amino acid sequences of the above-mentioned three linear polypeptide antigens which are fully complementary with the target antibody in terms of space structure and configuration.

Numerous studies prove that VEGFR1 is one tumor-associated antigen and can be expressed in different solid tumors. Therefore, VEGFR1 antibody can be considered as a natural anti-tumor antibody and can play the immunological surveillance role in human body and prevent the formation and progression of tumors. The technology of the present invention fills in the blank of quantitative detection of the natural anti-tumor antibody, and provides an important tool to develop new products in a plasma biologicals company and develop new measures against tumors in clinical medicine. For example, the plasma biologicals company can utilize the technology of the present invention to screen plasma to prepare gamma globulin enriched in VEGFR1 antibody for the clinical tumor prevention and treatment. Also, in clinics, patients at early stage of tumor after local treatment (for example, after surgery or radiotherapy) can be directly transfused with VEGFR1 antibody-enriched plasma screened by the technology of the present invention to enhance the immunosurveillance function thereof and prevent the tumor recurrence and metastasis. This is the application value of the technology of the present invention.

As shown in Table 1, the three antigen polypeptides applied to the detection of plasma VEFGR1 autoantibody by ELISA method is implemented in the form of high-purity products with the purity recommended being not less than 95%. The technical effect which can be achieved in the present invention is illustrated by the following technical verification using the sandwich contrast test pattern which is common in the art.

The present application also provides a method as defined in claims 3 and 4 for detecting plasma immune marker VEFGR1 autoantibody by using the three antigen polypeptides shown in Table 1. The method may optionally comprise the following steps of:
1. before operation, dissolving each of the antigen polypeptides into 5 mg/ml storage solution by using 65%-67% acetic acid, and then isometrically mixing and placing in a refrigerator at -20°C (error less than 2°C) for storage;
2. before start-up of the operation, firstly diluting the isometric mixed solution of the three antigen polypeptides shown in Table 1 with a coating buffer to 10-50 mcg/ml, wherein the coating buffer is 0.1 M phosphate buffer solution containing 0.15 M sodium chloride and 10 mM EDTA, and a pH value is 7.2-7.4;
3. coating a maleimide-activated 96-well plate (Thermo Scientific, U.S.A.), incubating at 4°C overnight, and rinsing with a washing solution for three times, wherein the washing solution is 0.1 M phosphate buffer solution containing 0.15 M sodium chloride and 0.1% TWEEN-20, and a pH value is 7.0-7.4; and
4. performing step-by-step sampling and analysis as follows:
   a. setting double holes in to-be-tested plasma sample and setting two negative control holes (NC,a reference being a negative control solution free of plasma VEFGR1 antibody, thereby obtaining index values of the three antigen polypeptides of the present invention shown in Table 1 in the negative environment free of VEFGR1 autoantibody, aiming to prove that the composition of the present invention is unlikely to appear a positive result in the case of having no VEFGR1 autoantibody) and two positive control holes (PC, a reference being a positive control solution containing VEFGR1 antibody standard, thereby obtaining index values of the three antigen polypeptides of the present invention shown in Table 1 at the level of VEFGR1 antibody standard reference content, aiming to prove that the composition of the present invention is likely to appear a positive result in the case of having VEFGR1 autoantibody, and further serving as a baseline for comparison to the index values of the to-be-tested plasma sample);
   b. diluting the to-be-tested plasma sample according to 1:200 by using an analysis solution, the analysis solution being the same as the antigen coating solution which is a 0.1 M phosphate buffer solution containing 0.15 M sodium chloride and 10 mM EDTA, and a pH value is 7.0-7.4, adding 100 µl into each hole, incubating at 25°C for 1-2 h, and then rinsing for three times;
   c. after according to the step b, using the analysis solution (which is a 0.1 M phosphate buffer solution containing 0.15 M sodium chloride and 10mM EDTA, and the pH value is 7.0-7.4), diluting horseradish peroxidase-labeled goat anti-human IgG (for verifying whether a detected substance in plasma is a specific antibody), the antibody having a working range of 1:10000-1:150000, adding 100 µl into each hole, and incubating at 25°C for 1-2 h;
   d. after using the aforementioned washing solution (which is 0.1M phosphate buffer solution containing 0.15M sodium chloride and 0.1% Tween-20, and the pH value of 7.0-7.4) to rinse for three times, adding 100 µl of a mixed solution of 3.3',5,5'-tetramethylbenzidine (TMB) and peroxidase into each hole, and keeping in dark place for 20-30 min at room temperature; and
   e. adding 50 µl of 10% sulfuric acid solution (10% H2SO4) as a stop solution into each hole, then detecting a value of optical density (OD) by using a microplate reader, wherein a detection wavelength is 450 nm and a reference wavelength is 630 nm, completing a detection process within 10 min after the stop solution is added, and accordingly quantitatively analyzing individual plasma VEGFR1 autoantibody level; and
5. performing an analysis on data obtained by the detection for each individual during group random sampling and analysis, and determining the relative level of VEGFR1 autoantibody in the plasma by using a positive sample ratio (PSR)., wherein a PSR calculation method is as follows: PSR=[VEGFR1 OD value-NC OD value]/[PC OD value-NC OD value]; and plotting by using a normality quartering method (Q-Q).

Three antigen polypeptides listed in Table 1 can be prepared into a simple and easy-to-use convenient test kit during practical application, which can be vacuum sealed and packaged with nonmetallic materials and can be stored for not less than six months at 4°C. Thus, the present application also provides a test box or kit as defined in claim 6 containing the three antigen polypeptides listed in Table 1. In short, after a maleimide-activated 96-well plate coated with a mixed solution of the three antigen polypeptides is dried in an oven at 45°C, the plate is vacuum sealed and packaged with nonmetallic packaging materials, and each reagent combination serves as a convenience test kit. A suggestion is given that all the three antigen polypeptides are articles not less than 95% in purity.

### BRIEF DESCRIPTION OF DRAWINGS

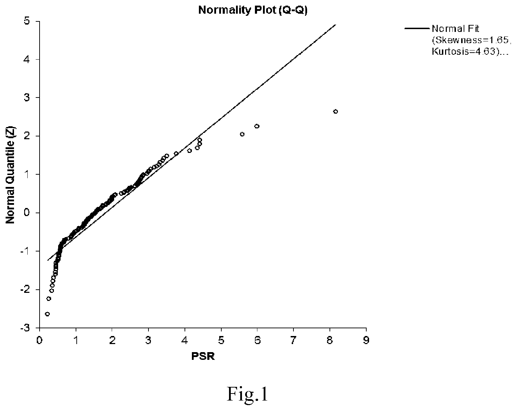

Fig. 1 is a quartile distribution graph of plasma VEGFR1 antibody concentration.

In the figure, the horizontal ordinate represents the plasma VEGFR1 antibody concentration indicative of PSR value, the longitudinal coordinate represents the quartile distribution range of plasma VEGFR1 antibody concentration, and the PSR value horizontally corresponding to 0 point is a median.

### DETAILED DESCRIPTION OF THE INVENTION

1. Sample collection is as follows: 121 plasma samples of normal adult human are collected, including plasma samples of 65 male cases and 56 female cases. All the blood sample providers are individuals determined by clinical physical examination to be free of having any type of tumors. Before operation, all the plasma samples are stored at -80°C, and verification shows that no plasma sample with repeated freezing and thawing (not more than 3 times) occurs during a storage period not longer than two years.
2. Sample detection is as follows: the plasma samples are thawed at 4°C, and the three antigen polypeptides listed in Table 1 used in the experiment are synthesized by UK SEVERN BIOTECH Co., Ltd., having a purity of 95%, and specific operation steps are as follows:
   (1) before operation, dissolving each of antigen polypeptides into 5.7 mg/ml storage solution by using 67% acetic acid, and then isometrically mixing and placing in a refrigerator at -20°C for storage;
   (2) before start-up of the operation, firstly diluting the mixed solution of the three antigen polypeptides with a coating buffer to 28.5 mcg/ml, wherein the coating buffer is 0.1 M phosphate buffer solution containing 0.15 M sodium chloride and 10mM EDTA, and the measured pH value is 7.2;
   (3) coating the maleimide-activated 96-well plate (Thermo Scientific, US), incubating at 4°C for 16.5 h overnight, and rinsing with a washing solution for three times, wherein the washing solution is 0.1 M phosphate buffer solution containing 0.15 M sodium chloride and 0.1% Tween-20, wherein the measured pH value is 7.3; and
   (4) performing step-by-step sampling and analysis as follows:
      a. setting double holes in to-be-tested plasma sample and additionally setting two negative control holes (NC, a reference being a human plasma negative control solution free of VEFGR1 antibody and provided by the company Sigma-Aldrich) and two positive control holes (PC, a reference is also a human plasma VEFGR1 antibody standard positive control solution provided by the company Sigma-Aldrich);
      b. diluting the plasma according to 1:200 by using an analytic solution, the analytic solution being the same as an antigen coating solution which is 0.1 M phosphate buffer solution containing 0.15M sodium chloride and 10mM EDTA, wherein the measured pH value is 7.2, adding 100 µl into each hole, and incubating at 25°C for 1.5 h;
      c. after using the aforementioned washing solution (which is 0.1 M phosphate buffer solution containing 0.15 M sodium chloride and 0.1% Tween-20, and the measured pH value is 7.2) to rinse for three times, diluting horseradish peroxidase-labeled goat anti-human IgG (provided by the company Sigma-Aldrich) by using the analytic solution , antibody having an working environment according to 1:285000, adding 100 µl into each hole, and incubating at 25°C for 1.5 h;
      d. after using the washing solution (which is 0.1 M phosphate buffer solution containing 0.15 M sodium chloride and 0.1% Tween-20, wherein the measured pH value is 7.2), rinsing for three times, adding 100 µl of 3,3,5,5-Tetramethylbenzidine (TMB) and a peroxidase mixed solution (provided by Life Technologies company) into each hole, and keeping in dark place for 25 min at room temperature; and
      e. adding 50 µl of 10% sulfuric acid solution (10% H2SO4) as stop solution into each hole, then detecting the value of optical density (OD) by using a microplate reader, wherein the detection wavelength is 450 nm and reference wavelength is 630 nm, and completing the detection within 10 minutes after stop solution is added.
3. Result analysis: the comparative analysis of VEGFR1 autoantibody distribution in target individuals is further carried out below in accordance with the experimental results. Upon analysis of the data obtained by the foregoing detection, the level of VEGFR1 autoantibody in plasma is determined by using a positive sample ratio (PSR), and PSR is calculated according to the following formula: PSR= [VEGFR1 OD value-NC OD value]/[PC OD value-NC OD value], and made into the following figure by normal quartering method (Q-Q). In the figure, the horizontal ordinate represents PSR value indicative of plasma VEGFR1 antibody concentration, the longitudinal coordinate represents the quartile distribution range of plasma VEGFR1 antibody concentration, and the PSR value horizontally corresponding to 0 point is the median.

As shown in Fig.1, the median PSR of plasma VEGFR1 antibody concentration is equal to 1.53, the skewness coefficient S is equal to 1.65, and the kurtosis coefficient K is equal to 4.63, which are statistically highly significant (w=0.89, p<0.0001). In the normal distribution fitting curve, the maximum value of rightward turning point is the threshold value (cut-off=3.8), namely, 8 out of totally 121 healthy adult plasma samples are rich in VEGFR1 autoantibody, and the number of individuals above the threshold value in this embodiment accounts for 6.6%. It can also be seen according to this embodiment that the level of VEGFR1 antibody in randomly selected healthy adult plasma samples is in skew distribution, wherein 8 plasma samples are rich in natural VEGFR1 autoantibody and valuable in clinical application.

In this embodiment, the synchronous negative contrast test proves that no false results exist in the present invention, and in the above figure, the horizontal ordinate represents the calculated values of PSR in individual blood samples, in reference to human plasma VEFGR1 antibody standard as positive control provided by Sigma-Aldrich company, which are marked to generate the above figure. In order to easily understand, in another aspect, the VEGFR1 autoantibody concentration (PSR value) can be distributed in 4 zones, and the proportion of cases falling within each antibody concentration zone in this embodiment is shown in the following table.

**Annexed table Distribution of VEGFR1 autoantibody in 121 healthy adult plasma samples**

| Cases | (%) | Antibody Concentration (PSR) |
|---|---|---|
| 34 | (28.1%) | 0.00-0.90 |
| 50 | (41.3%) | 0.91-2.30 |
| 29 | (24.0%) | 2.31-3.79 |
| 8 | (6.6%) | 3.80-8.60 |

In the above specific embodiment, the human plasma VEFGR1 antibody negative control solution and positive standard control solution provided by international authority biochemical reagent company Sigma-Aldrich are used, and the results of the sandwich contrast test prove that: the three linear antigen polypeptides designed in the present invention can specifically bond with VEFGR1 autoantibody in plasma, namely, the amino acid sequences of the three linear polypeptide antigens are effectively complementary with VEFGR1 antibody protein in terms of space structure and configuration, thus quantitatively determining the level of plasma VEFGR1 autoantibody in different individuals. As a result, a practical means for detecting plasma VEFGR1 autoantibody is obtained, and at the same time, the synthesis process for preparation of the three linear polypeptide antigens designed by the present invention is relatively simple and cost-reasonable, which lays a theoretical and practical basis for clinical application and development in tumor treatment with natural VEFGR1 autoantibody-rich plasma.
## Claims

1. A composition for detecting VEFGR1 autoantibody, comprising the following three antigen polypeptides:
H-DEGVYHCKATNQKGSVESSAYLTVQGTSDKSNLE-OH;
H-DLKLSCTVNKFLYRDVTWILLRTVNNRTMHYSI-OH;
H-ESGLSDVSRPSFCHSSCGHVSEGKRRFTYDHAEL-OH.

2. The composition according to claim 1, wherein the ratio of individual antigen polypeptides in the composition is 1:1:1.

3. A method for detecting VEFGR1 autoantibody in an individual, comprising the following steps: mixing equal amounts of the three antigen polypeptides according to claim 1 or 2, then coating a maleimide-activated 96-well plate, incubating at 4°C overnight, rinsing, and performing step-by-step sampling and analysis.

4. The method according to claim 3, wherein the step-by-step sampling and analysis comprises setting double holes in to-be-tested plasma sample while setting two negative control holes and two positive control holes, diluting plasma with an analysis fluid, diluting horseradish peroxidase-labeled goat anti-human IgG, rinsing, and adding 100 µl of 3,3',5,5'-tetramethylbenzidine (TMB) and peroxidase mixed solution into each hole, keeping in dark plate for 20-30 min at the room temperature, adding 50µl of 10% sulfuric acid solution (10% H₂SO₄) as a stop solution into each hole, and then, detecting the optical density (OD) value by using a microplate reader, wherein the detected wavelength is 450 nm, and the reference wavelength is 630 nm.

5. Use of the composition according to claim 1 or 2 *in vitro* for detection of a VEGFR1 autoantibody.

6. A test kit set prepared from the composition according to claim 1 or 2, **characterized in that** the three antigen polypeptides are respectively taken to be combined into the test kit set for later use after being independently packaged in vacuum by using nonmetallic medical packaging materials.

## Patentansprüche

1. Zusammensetzung zum Nachweisen von VEFGR1-Autoantikörper, die die folgenden drei Antigenpolypeptide umfasst:
H-DEGVYHCKATNQKGSVESSAYLTVQGTSDKSNLE-OH;
H-DLKLSCTVNKFLYRDVTWILLRTVNNRTMHYSI-OH;
H-ESGLSDVSRPSFCHSSCGHVSEGKRRFTYDHAEL-OH.

2. Zusammensetzung nach Anspruch 1, wobei das Verhältnis einzelner Antigenpolypeptide in der Zusammensetzung 1:1:1 beträgt.

3. Verfahren zum Nachweisen von VEFGR1-Autoantikörper in einem Individuum, das die folgenden Schritte umfasst: Mischen gleicher Mengen der drei Antigenpolypeptide nach Anspruch 1 oder 2, danach Beschichten einer maleimidaktivierten 96-Well-Platte, Inkubieren bei 4 °C über Nacht, Spülen und Durchführen einer Schritt-für-Schritt-Probennahme und -analyse.

4. Verfahren nach Anspruch 3, wobei die Schritt-für-Schritt-Probennahme und -analyse das Einrichten von Doppellöchern in einer zu testenden Plasmaprobe, während zwei Negativkontrolllöcher und zwei Positivkontrolllöcher eingerichtet werden, Verdünnen von Plasma mit einem Analysefluid, Verdünnen von mit Meerrettichperoxidase markiertem Ziegen-Anti-Humanes-IgG, Spülen und Hinzufügen von 100 µl einer gemischten Lösung von 3,3',5,5'-Tetramethylbenzidin (TBM) und Peroxidase in jedes Loch, Halten in dunkler Platte für 20 bis 30 min bei Raumtemperatur, Hinzufügen von 50 µl einer 10-%-Schwefelsäurelösung (10 % H₂SO₄) als Stopplösung in jedes Loch und danach Nachweisen des Werts der optischen Dichte (OD) unter Verwendung eines Mikroplattenlesers, wobei die nachgewiesene Wellenlänge 450 nm beträgt und die Referenzwellenlänge 630 nm beträgt.

5. Verwendung der Zusammensetzung nach Anspruch 1 oder 2 *in vitro* zum Nachweis eines VEFGR1-Autoantikörpers.

6. Testkitsatz, der aus der Zusammensetzung nach Anspruch 1 oder 2 hergestellt ist, **dadurch gekennzeichnet, dass** die drei Antigenpolypeptide jeweils verwendet werden, um in den Testkitsatz für eine spätere Verwendung kombiniert zu werden, nachdem sie unter Verwendung von nicht metallischen medizinischen Verpackungsmaterialien unabhängig unter Vakuum verpackt wurden.

## Revendications

1. Composition pour détecter un auto-anticorps contre VEFGR1, comprenant les trois polypeptides antigéniques suivants :
H-DEGVYHCKATNQKGSVESSAYLTVQGTSDKSNLE-OH ;
H-DLKLSCTVNKFLYRDVTWILLRTVNNRTMHYSI-OH ;
H-ESGLSDVSRPSFCHSSCGHVSEGKRRFTYDHAEL-OH.

2. Composition selon la revendication 1, dans laquelle le rapport des polypeptides antigéniques individuels dans la composition est de 1:1:1.

3. Procédé de détection d'un auto-anticorps contre VEFGR1 chez un individu, comprenant les étapes suivantes : mélanger des quantités égales des trois polypeptides antigéniques selon l'une des revendications 1 ou 2, puis revêtir une plaque à 96 puits activée par maléimide, incuber à 4°C pendant la nuit, rincer et réaliser un échantillonnage et une analyse pas à pas.

4. Procédé selon la revendication 3, dans lequel l'échantillonnage et l'analyse pas à pas comprend définir des doubles trous dans l'échantillon de plasma à tester tout en définissant deux trous de témoin négatif et deux trous de témoin positif, diluer le plasma avec un fluide d'analyse, diluer l'IgG anti-humain de chèvre marquée à la peroxydase de raifort, rincer et ajouter 100 µl d'une solution mixte de 3,3',5,5'-tétraméthylbenzidine (TMB) et de peroxydase dans chaque trou, laisser dans une plaque noire pendant 20 à 30 minutes à température ambiante, ajouter 50 µl de 10 % de solution d'acide sulfurique (10% H₂SO₄) en tant que solution d'arrêt dans chaque trou, puis, détecter la valeur de densité optique (DO) à l'aide d'un lecteur de microplaques, dans lequel la longueur d'onde détectée est 450 nm et la longueur d'onde de référence est 630 nm.

5. Utilisation de la composition selon l'une des revendications 1 ou 2 *in vitro* pour la détection d'un auto-anticorps contre VEGFR1.

6. Ensemble de kit de test préparé à partir de la composition selon l'une des revendications 1 ou 2, **caractérisé en ce que** les trois polypeptides antigéniques sont respectivement pris pour être combinés dans l'ensemble de kit de test pour une utilisation ultérieure après avoir été conditionnés indépendamment sous vide à l'aide de matériaux d'emballage médicaux non métalliques.
